Europäisches Patentamt

(19) European Patent Office

Office européen des brevets

(11) Publication number : **0 426 170 B1**

(12) **EUROPEAN PATENT SPECIFICATION**

(45) Date of publication of patent specification :
23.03.94 Bulletin 94/12

(51) Int. Cl.⁵ : **G01N 33/553**

(21) Application number : **90120939.5**

(22) Date of filing : **31.10.90**

(54) **Indirect agglutination immunoassay and apparatus therefor.**

(30) Priority : **31.10.89 JP 281895/89**
**21.12.89 JP 329556/89**

(43) Date of publication of application :
**08.05.91 Bulletin 91/19**

(45) Publication of the grant of the patent :
**23.03.94 Bulletin 94/12**

(84) Designated Contracting States :
**CH DE ES FR GB IT LI NL**

(56) References cited :
**EP-A- 0 351 857**
**US-A- 4 770 855**

(73) Proprietor : **FUJIREBIO INC.**
**6-7, Shimoochiai 4-chome Shinjuku-ku**
**Tokyo 161 (JP)**

(72) Inventor : **Saito, Tomo**
**207, Takatsu-ku Futago**
**Kawasaki-shi Kanagawa 213 (JP)**
Inventor : **Ikeda, Mikio**
**25-23, Sunagawa-cho 7-chome**
**Tachikawa-shi Tokyo (JP)**

(74) Representative : **Schwabe - Sandmair - Marx**
**Stuntzstrasse 16**
**D-81677 München (DE)**

## Description

## BACKGROUND OF THE INVENTION

The present invention relates to an indirect agglutination immunoassay and an apparatus therefor, and more particularly to an indirect agglutination immunoassay using an antigen-antibody reaction and magnetic particles or magnetic-material-containing particles and an apparatus for conducting the indirect agglutination immunoassay.

Indirect agglutination used in an immunoassay, in which the combining reaction by the antigen-antibody reaction is intensified by use of antigen- or antibody-bonded particles, is called "passive agglutination" or "reverse passive agglutination" and is widely used in practice in a simple immunoassay for a large number of test samples.

The immunoassay utilizing indirect agglutination has the advantages over conventional EIA (enzyme immunoassay) and RIA (radioimmunoassay) in that the assay is simple in operation and does not require any particular device for detecting the occurrence of the antigen-antibody reaction. However the immunoassay utilizing indirect agglutination has the drawbacks that it is extremely difficult to conduct the assay automatically, and it has not as yet been developed beyond the semi-automation stage.

There are two methods for forming detection patterns during indirect agglutination.

In one method, a particle-containing reagent is added to a diluted solution of a test sample placed in a "U" well or "V" well microplate, the mixture is stirred and then allowed to stand, and the occurrence of antigen-antibody reaction is detected from a sedimentation pattern of the particles of the reagent formed at the bottom of the well. Hereinafter this method is referred to as "the standing method".

In another method, a particle-containing reagent is added to a diluted solution of a test sample placed in a "U" well or "V" well microplate, and the mixture is stirred and then centrifuged to precipitate the particles onto the bottom of the well. The microplate is then inclined, so that the occurrence of an antigen-antibody reaction is detected from the slippage state of a coating of the precipitated particles at the bottom of the well of the microplate. Hereinafter this method is referred to as "the centrifugation method".

In the case where the occurrence of an antigen-antibody reaction is detected from the sedimentation pattern by the standing method, it is extremely difficult to detect the pattern automatically because the pattern is easily distorted by slight vibrations during the standing thereof. As a result, for instance, the pattern is deformed, the area of the pattern is decreased, and slippage of the pattern along the bottom of the well takes place. In addition, the standing method has the drawback that it takes up to about 0.5 to 3 hours before the pattern is formed in a suitable fashion for the assay, although the necessary time period for this of course depends upon the type of particles employed.

By contrast, in the case of the centrifugation method, the sedimentation can be finished within a few minutes by use of a centrifuge, and the pattern can be read after the microplate is slanted for several minutes. Furthermore, concern about the distortion of the pattern caused by vibrations applied thereto during the formation of the pattern is entirely unnecessary. However, it is difficult to perform the immunoassay automatically by use of a centrifuge in practice.

As test samples that can be used for the above-mentioned conventional standing method and centrifugation method, for instance, blood serum, urine, and other body fluids can be given. In conventional methods, the test samples are usually diluted and used. However, when whole blood is used without separating out of the blood corpuscles and blood serum, the sedimentation pattern tends to be centered at one point of the bottom of the well of the microplate, so that the blood components adversely affect the shape of the agglutination or sedimentation pattern. It is known that this will distort the results of the assay.

In other conventional immunoassays, such as the EIA (enzyme immunoassay) and RIA (radioimmunoassay), the separation of blood corpuscles and blood serum is conducted as a pre-processing step in order to avoid non-specific reactions.

EP-A-0 351 857 which is to be considered pursuant to Art. 54(3)EPC, discloses an indirect immunoagglutination assay carried out with magnetic particles but without inclination of the probes subsequent to precipitation of the agglutinate. US-A-4 770 885 describes an immunoagglutination assay which uses specially formed reaction vessels containing a plurality of inclination angles on their bottom surface.

## SUMMARY OF THE INVENTION

It is therefore a first object of the present invention to provide a simplified, automatic indirect agglutination immunoassay with an increased magnetic sedimentation rate.

A second object of the present invention is to provide an apparatus for performing the above agglutination immunoassay automatically.

According to the present invention, the first object of the present invention can be achieved by an magnetic agglutination immunoassay comprising the steps of (i) constructing an immunoassay system comprising a test sample, and a reagent comprising magnetic particles or magnetic-material containing particles to allow indirect agglutination to take place in a container, (ii) precipitating the magnetic particles or magnetic-material containing particles by the application of magnetic force, characterized by (iii) subsequently allowing the container to stand at an inclination, and (iv) judging the presence or absence of an immune reaction from the slippage state of the sedimented, agglutinated magnetic particles on the bottom of the container.

In the above magnetic agglutination immunoassay, the magnetic particles can be precipitated by use of either an electromagnet or a permanent magnet.

The second object of the present invention can be achieved by an apparatus comprising (a) a container which contains a test sample for immunoassays, and magnetic particles or magnetic-material-containing particles which can accept a reagent for immunoassay, (b) a magnetic sedimentation means for magnetically precipitating the components containing the magnetic particles at the bottom of the container, and (c) an inclination means for allowing the container to stand at an inclination.

## BRIEF DESCRIPTION OF THE DRAWINGS

In the drawings,

Fig. 1 is a block diagram of an apparatus for conducting the indirect agglutination immunoassay according to the present invention;

Fig. 2 is a diagram showing the relationship between the immunoassay according to the present invention and a conventional standing method with respect to the respective titers; and

Fig. 3 is a view of an example of the observation of test samples by use of a V-shaped well microplate.

Fig. 4 to 6 are the diagrams showing the relationship between the immunoassay according to the present invention and a conventional standing method with respect to the respective titers.

## DETAILED DESCRIPTION OF THE PREFERRED EMBODIMENTS

In the present invention, the magnetic particles are sedimented by magnetic force at the bottom of the container which is placed on a magnetic sedimentation acceleration base. The magnetic sedimentation acceleration base performs the same function of sedimenting the magnetic particles by force as a centrifuge does, so that the time required for an immunoassay can be reduced to 1/3 or less of the time required for the conventional standing method. Furthermore, the assay is completely unaffected by vibrations.

Examples of the magnetic particles or magnetic-material-containing particles to be contained in the reagent for use in the present invention include are magnetic particles, ferrimagnetic-material-containing gelatin particles as disclosed in Japanese Laid-Open Patent Application 59-195161, magnetic particles comprising a magnetic material coated with blood serum albumin or a polymeric material, and magnetic-material-containing polymer particles. As the materials for the magnetic particles or magnetic-material-containing particles, ferrimagnetic materials are preferably employed.

Test samples that can be tested by the magnetic agglutination immunoassay according to the present invention include blood serum, urine, body fluid, and whole blood. When whole blood is tested, only the components which contain the magnetic particles or magnetic-material-containing particles are sedimented by magnetic force at the bottom of the container. Therefore, the blood corpuscle components in the test sample spread throughout the test sample in the container, and only the components which contain the magnetic particles or magnetic-material-containing particles are selectively sedimented at one portion of the bottom of the container. Therefore even if whole blood is employed, the pattern formed by the components which contain the magnetic particles or magnetic-material-containing particles is not substantially affected by other components present in the test sample, and if any, the affect is in fact negligible.

When the container is colored red in its entirety by the blood corpuscle components and it is difficult to observe the sedimentation, the observation can be done easily by covering the container with a red-colored filter, made of, for example, glass, plastic film or cellophane.

As the containers for the test samples and reagents for use in the present invention, V-shaped or U-shaped, large or small, containers made of a plastic resin, such as polystyrene resin, ABS resin, or glass, can be employed.

In order to test a large number of test samples, it is preferable to use V-shaped well microplates made of polystyrene because the formed patterns can be clearly observed.

As mentioned previously, the apparatus for conducting the magnetic agglutination immunoassay according to the present invention comprises (a) a container for holding a test sample for immunoassay, and magnetic particles which can accept a reagent for immunoassay, (b) a magnetic sedimentation means for magnetically precipitating the components containing the magnetic particles at the bottom of the container, and (c) an inclination means for allowing the container to stand at an inclination.

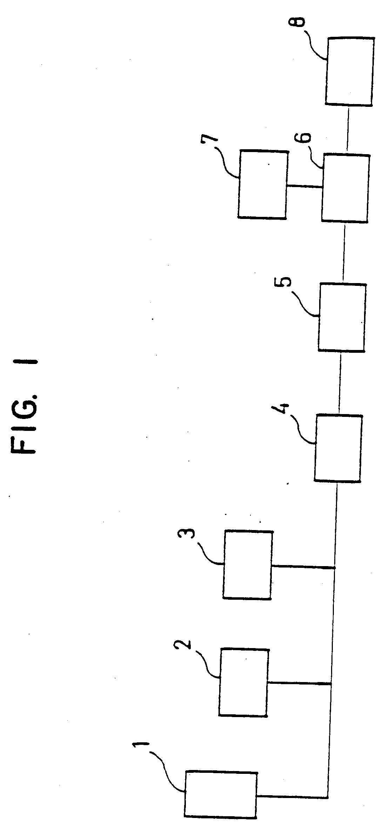

An example of the apparatus according to the present invention, which is automized for use in practice, will now be explained with reference to Fig. 1, which is a diagram of the apparatus and is given for illustration of the invention and is not intended to be limiting thereof.

In Fig. 1, reference numeral 1 indicates a microplate supply device comprising, for instance, a number of V-shaped wells on a microplate (not shown), which serve as the above-mentioned container for holding a test sample for immunoassay, and magnetic particles which can accept a reagent for immunoassay; reference numeral 2, a test sample supply device for supplying a test sample; reference numeral 3, a reagent supply device for supplying a reagent comprising magnetic particles or magnetic-material-containing particles; reference numeral 4, a stirrer for stirring the test sample and the reagent; reference numeral 5, a sedimentation acceleration device for sedimenting the magnetic particles or magnetic-material-containing particles at the bottom of the microplate; reference numeral 6, an inclination device for allowing the microplate supply device 1 to stand at an inclination; reference numeral 7, a pattern read apparatus for reading or detecting the slippage state of the sedimented particles on the bottom caused by the inclination of the microplate supply device 1; and reference numeral 8, a microplate recovery device for recovering the used microplates.

Example 1 [Preparation of gelatin particle containing anti-human alpha($\alpha$)-fetoprotein (AFP) sensitized ferricolloid]

Gelatin particles containing anti-human AFP sensitized ferricolloid were prepared by application of anti-human AFP antibody (rabbit, DACO) to ferricolloid-containing gelatin particles having an average particle size of about 3 microns disclosed in Japanese Laid-Open Patent Application 59-195161 in accordance with a method by Barnard et al. (Clin. Chem., $\underline{27}$ (6) 832 (1981)).

Example 2 [Assay of AFP in Blood Serum]

Each of a series of blood serum test samples containing AFP at a different concentration was diluted by 10 times with a blood serum dilution liquid.

Each of the thus diluted blood serum test samples was placed in the V-shaped wells of a microplate. To each diluted test sample placed in the V-shaped well of the microplate was added 25 $\mu$l of a dispersion of the gelatin particles containing anti-human AFP sensitized ferricolloid prepare in Example 1 with a concentration of 0.09%. The mixture was stirred for 10 minutes.

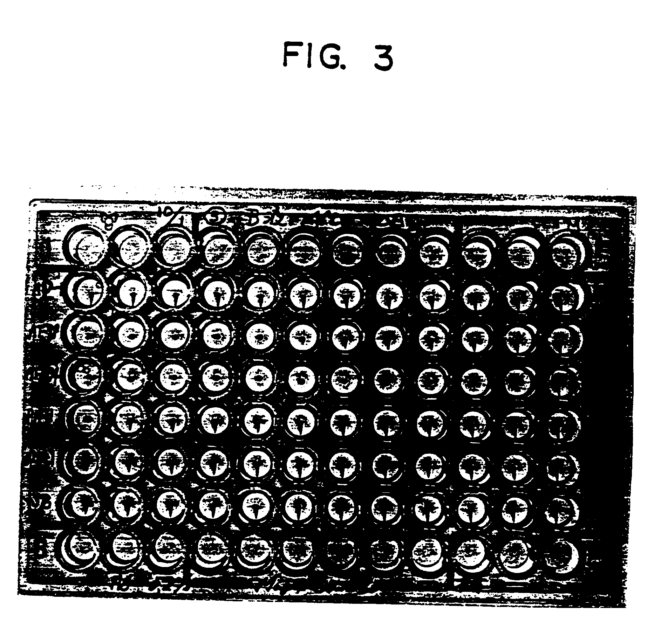

The microplate was allowed to stand on a magnet-including sedimentation acceleration base for 5 minutes. The microplate was then removed from the sedimentation acceleration base and placed on a pattern reading base free from magnetic force and allowed to stand thereon at an inclination of about 70° for one minute to observe the slippage state of a coating of the sedimented particles at the bottom of each well, whereby the presence or absence of the immune reaction was judged. When slippage was observed in the coating of the sedimented particles, the immune reaction was judged not to have occurred, that is, the immune reaction was negative, while when no slippage of the coating of the sedimented particles was observed, the immune reaction was judged to have occurred, that is, the immune reaction was positive. Fig. 3 shows a view of an example of an observation of an example of such test samples obtained by use of a V-shaped well microplate.

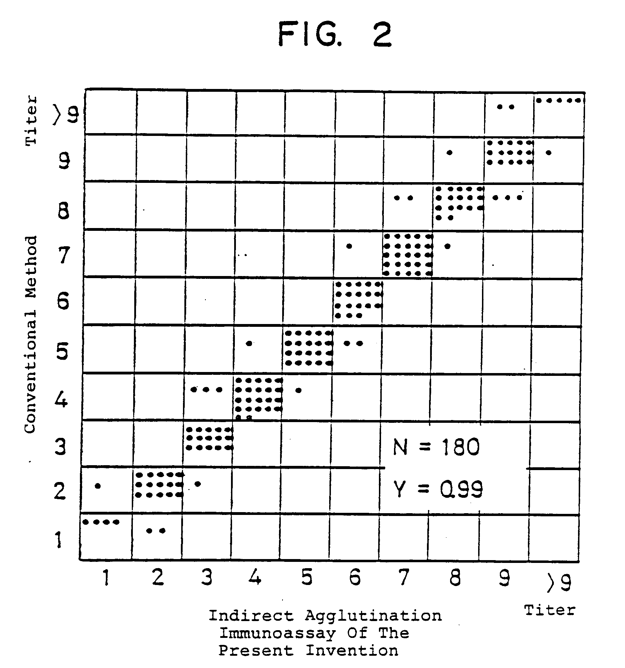

Fig. 2 shows the relationship between the above-mentioned immunoassay according to the present invention and a conventional standing method using a commercially available reagent for the assay of alpha($\alpha$)-fetoprotein (Trademark "Serodia AFP mono" made by Fujirebio Inc.) with respect to the respective titers.

Example 3 [Assay of AFP in Blood Serum]

A blood serum test sample containing AFP was diluted by 10 times with a blood serum dilution liquid. 50 $\mu$l of the thus diluted blood serum test sample was placed in a first V-shaped well of a microplate. 25 $\mu$l of the blood serum dilution liquid was placed in each of a second well through an eighth well of the microplate. 25 $\mu$l of the diluted blood serum test sample was taken from the first well, and by use of the diluted blood serum test sample, a 2n dilution was sequentially performed from the second well through the eighth well. To each diluted test sample placed in the V-shaped wells of the microplate was added 25 $\mu$l of a dispersion of the gelatin particles containing anti-human AFP sensitized ferricolloid prepared in Example 1 at a concentration of 0.14%. The mixture was stirred for 3 minutes. The microplate was allowed to stand on a magnet-including sedimentation acceleration base for 3 minutes. The microplate was then removed from the sedimentation acceleration base and placed on a pattern reading base free from the effect of magnetic force and allowed to stand thereon at an inclination of about 45° for one minute to observe the slippage state of a coating of the sedimented particles on the bottom of each well, whereby the presence or absence of the immune reaction was judged in the same manner as in Example 2. The results are shown in TABLE 1.

Example 4 [Assay of AFP in Whole Blood]

A blood serum test sample containing AFP was diluted by 5 times with a blood serum dilution liquid. To this diluted blood serum test sample was added a whole blood collected from a man in good health in an amount equal to the amount of the diluted blood serum test sample, whereby a test sample was prepared.

50 µl of the thus prepared test sample was placed in a first V-shaped well of a microplate. 25 µl of the blood serum dilution liquid was placed in each of a second well through an eighth well of the microplate. 25 µl of the test sample was taken from the first well, and by use of the diluted test sample, a 2n dilution was sequentially performed from the second well through the eighth well. To each diluted test sample placed in the V-shaped wells of the microplate was added 25 µl of a dispersion of the gelatin particles containing anti-human AFP sensitized ferricolloid prepared in Example 1 at a concentration of 0.14%. The mixture was stirred for 3 minutes.

The microplate was allowed to stand on a magnet-including sedimentation acceleration base for 3 minutes. The microplate was then removed from the sedimentation acceleration base and placed on a pattern reading base free from the effect of magnetic force and allowed to stand thereon at an inclination of about 45° for one minute to observe the slippage state of a carpet of the sedimented particles from the bottom of each well, whereby the presence or absence of the immune reaction was judged. The results are shown in TABLE 1.

### TABLE 1

| Final Dilution Ratio (Blood Serum) | Example 3 Judgement | Example 4 | | |
|---|---|---|---|---|
| | | Final Dilution Ratio (Whole Blood) | Judgement | |
| 1 : 20 | + | 1 : 4 | +* | |
| 1 : 40 | + | 1 : 8 | +* | |
| 1 : 80 | + | 1 : 16 | + | |
| 1 : 160 | + | 1 : 32 | + | |
| 1 : 320 | − | 1 : 64 | − | |
| 1 : 640 | − | 1 : 128 | − | |
| 1 : 1280 | − | 1 : 256 | − | |
| 1 : 2560 | − | 1 : 512 | − | |

+ : Positive

− : Negative

−*: Difficult to judge

Example 5 [Assay of AFP in Blood Serum]

Each of a series of blood serum test samples containing AFP at a different concentration was diluted by 10 times with a blood serum dilution liquid.

Each of the thus diluted blood serum test samples was placed in the V-shaped wells of a microplate. To each diluted test sample placed in the V-shaped well of the microplate was added 25 μl of a dispersion of the gelatin particles containing anti-human AFP sensitized ferricolloid prepared in Example 1 at a concentration of 0.14%. The mixture was stirred for 5 minutes.

The microplate was allowed to stand on a magnet-including sedimentation acceleration base for 5 minutes. The microplate was then removed from the sedimentation acceleration base and placed on a pattern reading base free from the effect of magnetic force and allowed to stand thereon at an inclination of about 45° for one minute to observe the slippage state of a coating of the sedimented particles at the bottom of each well. One minute later, the presence or absence of the immune reaction was judged. As mentioned previously when slippage was observed in the coating of the sedimented particles, the immune reaction was judged not to have occurred, that is, the immune reaction was negative, while when no slippage of the coating of the sedimented particles was observed, the immune reaction was judged to have occurred, that is, the immune reaction was positive.

Example 6 [Assay of AFP in Blood Serum]

A blood serum test sample containing AFP was diluted by 10 times with a blood serum dilution liquid. 50 μl of the thus diluted blood serum test sample was placed in a first V-shaped well of a microplate. 25 μl of the blood serum dilution liquid was placed in each of a second well through an eighth well of the microplate. 25 μl of the diluted blood serum test sample, was taken from the first well, and by use of the diluted blood serum test sample, a 2n dilution was sequentially performed from the second well through the eighth well.

To each diluted test sample placed in the V-shaped wells of the microplate was added 25 μl of a dispersion of the gelatin particles containing anti-human AFP sensitized ferricolloid prepared in Example 1 at a concentration of 0.14%. The mixture was stirred for 5 minutes.

The microplate was allowed to stand on a magnet-including sedimentation acceleration base for 5 minutes. The microplate was then removed from the sedimentation acceleration base and placed on a pattern reading base free from the effect of magnetic force and allowed to stand thereon at an inclination of about 45° for one minute to observe the slippage state of a coating of the sedimented particles at the bottom of each well, whereby the presence or absence of the immune reaction was judged in the same manner as in Example 5. The results were the same as in Example 3 shown in TABLE 1.

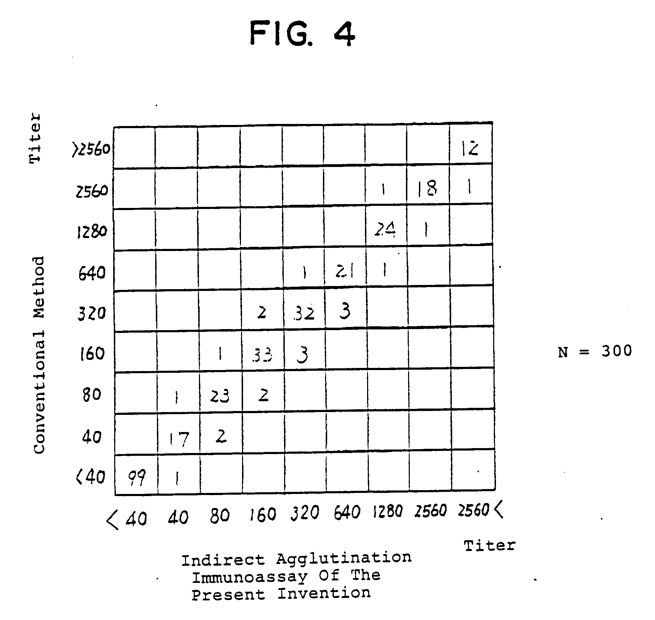

Fig. 4 shows the relationship between the above-mentioned immunoassay according to the present invention and a conventional standing method using a commercially available reagent for the detection of alpha(α)-fetoprotein (Trademark "Serodia AFP mono" made by Fujirebio Inc. with respect to the respective titers.

Example 7 [Assay of AFP in Whole Blood]

A blood serum test sample containing AFP was diluted by 5 times with a blood serum dilution liquid. To this diluted blood serum test sample was added a whole blood collected from a man in good health in an amount equal to the amount of the diluted blood serum test sample, whereby a test sample was prepared.

50 μl of the thus prepared test sample was placed in a first V-shaped well of a microplate. 25 μl of the blood serum dilution liquid was placed in each of a second well through an eighth well of the microplate. 25 μl of the test sample was taken from the first well, and by use of the test sample, a 2n dilution was sequentially performed from the second well through the eighth well.

To each diluted test sample placed in the V-shaped wells of the microplate was added 25 μl of a dispersion of the gelatin particles containing anti-human AFP sensitized ferricolloid prepared in Example 1 at a concentration of 0.14%. The mixture was stirred for 5 minutes.

The microplate was allowed to stand on a magnet-including sedimentation acceleration base for 5 minutes. The microplate was then removed from the sedimentation acceleration base and placed on a pattern reading base and allowed to stand thereon at an inclination of about 45° for one minute to observe the slippage state of a coating of the sedimented particles at the bottom of each well, whereby the presence or absence of the immune reaction was judged in the same manner as in Example 5. The results were the same as in Example 4 shown in TABLE 1.

Example 8 [Preparation of gelatin particle containing ATLV antigen sensitized ferricolloid]

Gelatin particles containing ATLV antigen sensitized ferricolloid were prepared by application of ATLV antigen to ferricolloid-containing gelatin particles having an average particle size of about 2.5 microns disclosed in Japanese Laid-Open Patent Application 59-195161 in accordance with a conventional method disclosed in Japanese Laid-Open Patent Application 60-44870.

Example 9 [Assay of ATLV antibody in Blood Serum]

25 $\mu$l of a blood serum dilution liquid was placed in each of wells of a microplate. 25 $\mu$l of a blood serum test sample was added to the blood serum dilution liquid in a first well. From the first well, 25 $\mu$l of the diluted blood diluted blood serum test sample was taken. By use of the diluted blood test sample, a 2n dilution was sequentially performed from the second well through the twelfth well. To each diluted test sample placed in the V-shaped wells of the microplate was added 25 $\mu$l of a dispersion of the gelatin particle containing the ATLV antigen sensitized ferricolloid prepared in Example 8 at a concentration of 0.2 %. The mixture was stirred for 5 minutes.

The microplate was allowed to stand on a magnet-including sedimentation acceleration base for 30 seconds. The microplate was then removed from the sedimentation acceleration base and placed on a pattern reading base free from the effect of magnetic force and allowed to stand thereon at an inclination of about 60° for one minute to observe the slippage state of a coating of the sedimented particles at the bottom of each well, whereby the presence or absence of the immune reaction was judged in the same manner as in Example 5.

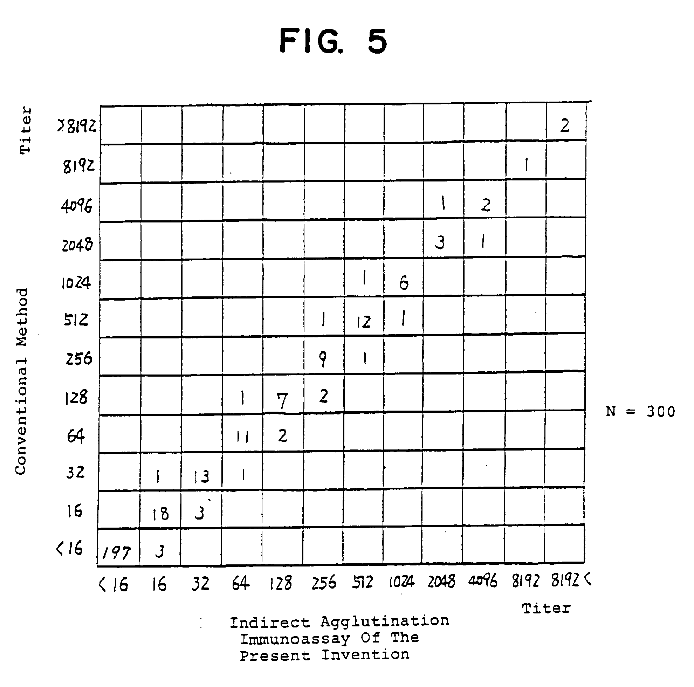

Fig. 5 shows the relationship between the above-mentioned immunoassay according to the present invention and a conventional standing method using a commercially available reagent for the detection of ATLV antibody (Trademark "Serodia ATLV antibody detection agent" made by Fujirebio Inc.) with respect to the respective titers.

Example 10 [Preparation of gelatin particle containing HIV antigen sensitized ferricolloid]

Gelatin particles containing HIV antigen sensitized ferricolloid were prepared by application of HIV antigen to ferricolloid-containing gelatin particles having an average particle size of about 2.5 microns disclosed in Japanese Laid-Open Patent Application 59-195161 in accordance with a conventional method disclosed in Japanese Laid-Open Patent Application 62-182662.

Example 11 [Assay of HIV antibody in Blood Serum]

75 $\mu$l of a blood serum dilution liquid was placed in each of wells of a microplate. 25 $\mu$l of a blood serum test sample was added to the blood serum dilution liquid in a first well. From the first well, 25 $\mu$l of the diluted blood serum test sample was taken. By use of the diluted blood test sample, a 2n dilution was sequentially performed from the second well through the twelfth well. To each diluted test sample placed in the V-shaped wells of the microplate was added 25 $\mu$l of a dispersion of the gelatin particle containing HIV antigen sensitized ferricolloid prepared in Example 10 at a concentration of 0.2 %. The mixture was stirred for 5 minutes.

The microplate was allowed to stand on a magnet-including sedimentation acceleration base for 30 seconds. The microplate was then removed from the sedimentation acceleration base and placed on a pattern reading base and allowed to stand thereon at an inclination of about 60° for one minute to observe the slippage state of a coating of the sedimented particles at the bottom of each well, whereby the presence or absence of the immune reaction was judged in the same manner as in Example 5.

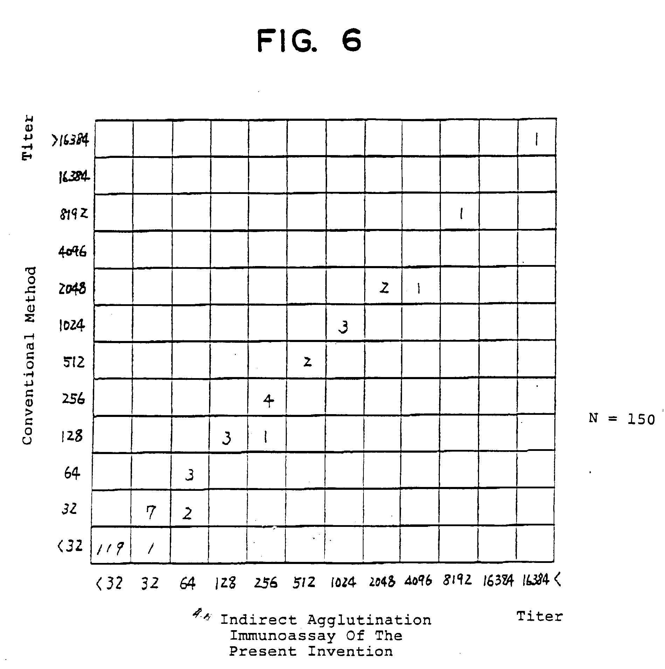

Fig. 6 shows the relationship between the above-mentioned immunoassay according to the present invention and a conventional standing method using a commercially available reagent for the detection of HIV antibody (Trademark "Serodia HIV antibody detection agent" made by Fujirebio Inc. with respect to the respective titers.

Thus, according to the present invention, the indirect agglutination immunoassay can be easily automized in sharp contrast to the conventional method by using a centrifuge. Furthermore, the indirect agglutination immunoassay according to the present invention can be conducted in a shorter time as compared with the conventional standing method, without being affected by environmental conditions such as vibrations. In addition, according to the present invention, whole blood can be employed as a test sample without any problems, which is impossible in the case of the conventional method using a centrifuge. Finally, there is a conspicuous correlation between the indirect agglutination immunoassay according to the present invention and the conventional standing method, and this assay can be applied not only to blood serum, urine and other body fluids, but also to whole blood, so that the conventional standing method can be sufficiently replaced by the indirect agglutination immunoassay according to the present invention and the present invention provides a simpler immunoassay method.

## Claims

1. An indirect agglutination immunoassay comprising the steps of:

constructing an immunoassay system comprising a test sample, and a reagent comprising magnetic particles or magnetic-material containing particles to allow indirect agglutination to take place in a container,

precipitating said magnetic particles or magnetic-material containing particles by the application of magnetic force, characterized by subsequently

allowing said container to stand at an inclination, and

judging the presence or absence of an immune reaction from the slippage state of the sedimented, agglutinated magnetic particles from the bottom of said container.

2. An apparatus for conducting an indirect agglutination immunoassay comprising:

(a) a container which contains a test sample for immunoassay, and magnetic particles or magnetic-material-containing particles which contain an immunoassay reagent,

(b) a magnetic sedimentation means for magnetically precipitating the components containing said magnetic particles at the bottom of said container, and

(c) an inclination means for allowing said container to stand at an inclination.

## Patentansprüche

1. Indirektes Agglutinationsimmunnachweisverfahren, bei dem man

ein Immunnachweissystem aufbaut, das eine Testprobe und ein Reagenz umfaßt, das magnetische Teilchen oder magnetisches Material enthaltende Teilchen enthält, um die indirekte Agglutinierung in einem Behälter ablaufen zu lassen,

diese magnetischen Teilchen oder magnetisches Material enthaltenden Teilchen durch Anwendung von Magnetkraft ausfällt, dadurch gekennzeichnet, daß man anschließend

den Behälter in einer Schräglage stehenläßt und

das Vorhandensein bzw. das Fehlen einer Immunreaktion nach dem Verrutschungszustand der sedimentierten agglutinierten magnetischen Teilchen vom Boden des Behälters beurteilt.

2. Apparat zur Durchführung eines indirekten Agglutinationsimmunnachweisverfahrens umfassend:

(a) einen Behälter, der eine Testprobe für das Immunnachweisverfahren und magnetische Teilchen oder magnetisches Material enthaltende Teilchen, die ein Reagenz für das Immunnachweisverfahren enthält, enthält,

(b) eine magnetische Abscheidevorrichtung für die magnetische Ausfällung der Komponenten, die die magnetischen Teilchen enthalten, am Boden des Behälters und

(c) eine Vorrichtung zur Schrägstellung, damit der Behälter schräg stehen kann.

## Revendications

1. Un essai immunologique d'agglutination indirecte, comprenant les étapes de :

établissement d'un système d'essai immunologique comprenant un échantillon d'essai et un réactif comprenant des particules magnétiques ou des particules contenant une matière magnétique pour permettre qu'une agglutination indirecte se produise dans un récipient,

précipitation desdites particules magnétiques ou desdites particules contenant une matière magnétique par application d'une force magnétique,

caractérisé en ce que ultérieurement

on laisse ledit récipient reposer en position inclinée et

on évalue la présence ou l'absence d'une réaction immune à partir de l'état de glissement des particules magnétiques agglutinées sédimentées a partir du fond dudit récipient.

2. Un appareil pour effectuer un essai immunologique d'agglutination indirecte, comprenant :

(a) un récipient qui contient un échantillon d'essai pour l'essai immunologique et des particules magnétiques ou des particules contenant une matière magnétique qui contiennent un réactif d'essai immunologique,

(b) un dispositif de sédimentation magnétique pour précipiter magnétiquement les composants contenant lesdites particules magnétiques au fond dudit récipient et

(c) un dispositif d'inclinaison pour permettre que ledit récipient repose en position inclinée.

EP 0 426 170 B1

# FIG. I

# FIG. 2

Indirect Agglutination
Immunoassay Of The
Present Invention

N = 180

Y = 0.99

FIG. 3

# FIG. 4

Conventional Method — Titer

| Titer | <40 | 40 | 80 | 160 | 320 | 640 | 1280 | 2560 | 2560< |
|-------|-----|-----|-----|-----|-----|-----|------|------|-------|
| >2560 | | | | | | | | | 12 |
| 2560 | | | | | | | 1 | 18 | 1 |
| 1280 | | | | | | | 24 | 1 | |
| 640 | | | | | 1 | 21 | 1 | | |
| 320 | | | | 2 | 32 | 3 | | | |
| 160 | | | 1 | 33 | 3 | | | | |
| 80 | | 1 | 23 | 2 | | | | | |
| 40 | | 17 | 2 | | | | | | |
| <40 | 99 | 1 | | | | | | | |

N = 300

Indirect Agglutination
Immunoassay Of The
Present Invention

Titer

# FIG. 5

Titer

| Conventional Method \ Titer | <16 | 16 | 32 | 64 | 128 | 256 | 512 | 1024 | 2048 | 4096 | 8192 | 8192< |
|---|---|---|---|---|---|---|---|---|---|---|---|---|
| >8192 | | | | | | | | | | | | 2 |
| 8192 | | | | | | | | | | | 1 | |
| 4096 | | | | | | | | | 1 | 2 | | |
| 2048 | | | | | | | | | 3 | 1 | | |
| 1024 | | | | | | | | 1 | 6 | | | |
| 512 | | | | | | | 1 | 12 | 1 | | | |
| 256 | | | | | | | 9 | 1 | | | | |
| 128 | | | | | 1 | 7 | 2 | | | | | |
| 64 | | | | | 11 | 2 | | | | | | |
| 32 | | 1 | 13 | 1 | | | | | | | | |
| 16 | | 18 | 3 | | | | | | | | | |
| <16 | 197 | 3 | | | | | | | | | | |

N = 300

Indirect Agglutination
Immunoassay Of The
Present Invention

Titer

# FIG. 6

Indirect Agglutination Immunoassay Of The Present Invention

N = 150